# EUROPEAN PATENT APPLICATION

(11) **EP 4 595 989 A1**
(43) Date of publication of application: **06.08.2025**
(21) Application number: 23870086.8
(22) Date of filing: 25.08.2023
(51) Int. Cl.: A61L 27/18, A61L 27/52, A61L 27/50, A61L 27/12, A61L 27/54, A61L 27/20

(54) **POLYCAPROLACTONE MICROSPHERE PRE-DISPERSION COMPOSITION AND POLYCAPROLACTONE INJECTABLE GEL PREPARED FROM SAME**

(30) Priority: 30.09.2022 CN 202211205858
(71) Applicant: Sinclair Pharmaceuticals Limited, Chester CH4 9QT (GB)
(72) Inventor: DONG, Xijian, Hangzhou, Zhejiang 310011 (CN); ZHANG, Hui, Hangzhou, Zhejiang 310011 (CN)
(74) Representative: Nederlandsch Octrooibureau
(86) International application number: PCT/CN2023/114949
(87) International publication number: WO 2024/066843

(57) **Abstract**

Disclosed in the present invention are a polycaprolactone microsphere pre-dispersion composition and a polycaprolactone injectable gel prepared from same. **In** the polycaprolactone microsphere pre-dispersion composition, polycaprolactone microspheres can continue to maintain their integrity as complete spheres with smooth surfaces and good dispersity, and have a reduced degradation rate; and bubbles can be reduced in a mixing process, thereby helping to ensure the stability of product quality, and reducing the occurrence probability of a nodule and a granuloma caused by uneven dispersion after the microspheres are injected into a body. The application value of the polycaprolactone injectable gel prepared from the polycaprolactone microsphere pre-dispersion composition is higher.

## Description

### Technical Field

The present invention relates to the technical field of medical cosmetology or medical preparations, in particular to a polycaprolactone microsphere pre-dispersed composition and a polycaprolactone injectable gel prepared from same.

### Background Art

Polycaprolactone (PCL) was first synthesized in the 1930s, being a linear aliphatic polyester obtained by ring-opening polymerization of ε-caprolactone. It is a hydrophobic, semi-crystalline polymer, which is in a rubber state at room temperature; it has good thermal stability, and its decomposition temperature is much higher than that of other polyesters. The products of PCL degradation are CO₂ and H₂O, which are non-toxic to the human body. Due to its specific physicochemical and mechanical properties, viscoelasticity and shapability, PCL-based products of various shapes and lifetimes are produced, specifically depending on the biodegradation kinetics thereof. PCL has already been safely used for more than 70 years in the biomedical field; its latest applications mostly involve making it into microspheres for use as medical aesthetic fillers of materials implanted in the body, to stimulate the production of collagen. PCL-based collagen simulating agents consist of PCL microspheres suspended in a carboxymethyl cellulose gel carrier, and are able to provide an immediate and lasting filling-out effect. The biocompatibility and form of PCL microspheres implanted into the collagen fibres produced help to create a unique 3D support, in order to realize a lasting effect.

As demand in the medical aesthetics market steadily grows, the market prospects of PCL have broadened. This is primarily by making PCL into microspheres; its scope of application in the form of gel consists of the forehead, the nasolabial folds, the middle of the face, the nose, the lower jaw and the hands. It remains effective for a maximum of 24 months, and its safety and effectiveness have already been confirmed in many clinical tests. Generally, the diameter of PCL injectable microspheres may be in the range of 25 - 50 µm. Microspheres of such a size are just right for avoiding phagocytosis by macrophages and remaining in bodily tissue. Both their size and shape have been proven to have a major influence on tissue response. Their shape - round or irregular - determines the degree of the response. PCL microspheres are regular spheres with a smooth surface, and these features are known as being the optimum features for minimizing an inflammatory response. If the sphere that is formed is smoother, this is better for subcutaneous injection. Microspheres with a high level of smoothness stimulate the enveloping of fibre-forming cells beneath wrinkles one by one, while also being able to reduce the occurrence of granulomas and reactions to foreign objects. Generally, for injection of microspheres, prepared smooth-surfaced PCL microspheres (volume ratio 30%) are uniformly suspended in 70% CMC gel.

Regarding the preparation of smooth-surfaced PCL microspheres, various methods for preparing smooth PCL microspheres have already been disclosed in the prior art. For example, CN104001209B has disclosed filtering, washing and drying obtained microspheres, then dispersing them in CMC gel. In the preparation process, to avoid solidification of the PCL solution in a stirred aqueous medium, the conditions employed include adding the DCM solution of PCL over a long period of time, and a long DCM evaporation time, so that the dispersed PCL particles harden. That document also investigates the Hunter method (Example 14 in US2003/0157187A), the Erneta and Wu method (Examples in EP1872803A1), etc., and summarizes the effects of surfactants in water and DCM, as well as surfactant viscosity and stirring speed, etc., on particle shape and particle surface smoothness. CN109998997B has disclosed that microspheres prepared by membrane emulsification have an average microsphere diameter of 50 µm after filtering, washing and drying; in terms of appearance and form, they have a smooth surface and good form. However, if it is desired to disperse prepared, dry microspheres into gel and maintain surface smoothness in the gel, further research into microsphere dispersion processes and gels is needed. Due to the fact that some air will be adsorbed on the surface before PCL microspheres are mixed into CMC gel, the problem of liquid and gas starting to contend for the microsphere surface will arise in the process of mixing evenly, with the result that bubbles are difficult to remove from the microsphere surface. In addition, because CMC gel has high viscosity, PCL microspheres are difficult to disperse and readily form bubbles in the process of mixing evenly. Therefore, there is a need for a novel approach to resolve the abovementioned problems in the process of mixing evenly. Furthermore, research has found that hydrolysis of PCL microspheres is related to penetration of microspheres by water. When water penetrates into microspheres, an overall degradation process will take place, with the result that ester bonds throughout the polymer matrix are gradually hydrolysed from the inside. Thus, further research is needed into microsphere dispersion processes and the gel foundation, to encourage the prepared PCL microspheres to still have a smooth surface in the gel, while hydrolysis of PCL can be further reduced.

### Summary of the Invention

To overcome the shortcomings in the prior art, the present invention provides a polycaprolactone (PCL) microsphere pre-dispersed composition, and an injectable gel containing a high concentration of polycaprolactone, said gel being prepared from the composition. The inventors unexpectedly discovered through research that a step of mixing evenly or re-dispersion after drying of PCL microspheres was a key factor influencing injectable PCL microsphere dispersion, bubble formation and smoothness. In addition, the inventors also unexpectedly found that compared with PCL microsphere dispersion media commonly used in the prior art - purified water, distilled water, normal saline, etc. - the dispersion medium and gel matrix chosen in the present invention can effectively reduce PCL degradation; the retention of the smooth spherical form of the microspheres helps to reduce the rate of microsphere degradation.

In a first aspect of the present invention, a polycaprolactone microsphere pre-dispersed composition is provided, comprising polycaprolactone microspheres and a phosphate buffer.

Specifically, the phosphate buffer comprises a dibasic phosphate salt and/or a monobasic phosphate salt; more specifically, the phosphate buffer comprises a dibasic phosphate salt and a monobasic phosphate salt.

Specifically, the dibasic phosphate salt is selected from one or more of disodium hydrogen phosphate, dipotassium hydrogen phosphate and diammonium hydrogen phosphate.

Specifically, the monobasic phosphate salt is selected from one or more of sodium dihydrogen phosphate, potassium dihydrogen phosphate or ammonium dihydrogen phosphate.

In some embodiments of the present invention, the dibasic phosphate salt is disodium hydrogen phosphate, and the monobasic phosphate salt is sodium dihydrogen phosphate.

In other embodiments of the present invention, the dibasic phosphate salt is disodium hydrogen phosphate, and the monobasic phosphate salt is potassium dihydrogen phosphate.

In other embodiments of the present invention, the dibasic phosphate salt is dipotassium hydrogen phosphate, and the monobasic phosphate salt is potassium dihydrogen phosphate.

In other embodiments of the present invention, the dibasic phosphate salt is dipotassium hydrogen phosphate, and the monobasic phosphate salt is sodium dihydrogen phosphate.

Specifically, the phosphate buffer may further comprise a pH regulator, e.g. one or more of hydrochloric acid, sodium hydroxide and potassium hydroxide, etc.

Specifically, the phosphate buffer has a pH of 6.0 - 8.0 (e.g. 6.0, 6.2, 6.4, 6.5, 6.6, 6.8, 7.0, 7.2, 7.4, 7.5, 7.6, 7.8, 8.0), in particular 6.5 - 8.0, preferably 7.0 - 7.5.

Specifically, the phosphate buffer may further comprise an osmotic pressure regulator, e.g. one or more of potassium chloride, sodium chloride and glycerol, etc.

Specifically, the phosphate buffer has an osmotic pressure of 300 - 700 mOsm/kg (e.g. 300, 350, 400, 450, 500, 550, 60 mOsm/kg), in particular 500 - 650 mOsm/kg, preferably 550 - 650 mOsm/kg.

In some preferred embodiments of the present invention, the phosphate buffer comprises disodium hydrogen phosphate and sodium dihydrogen phosphate, and has a pH of 6.0 - 8.0 (in particular 6.5 - 7.5, e.g. 7.2) and an osmotic pressure of 500 - 650 mOsm/kg; more specifically, the phosphate buffer consists of disodium hydrogen phosphate, sodium dihydrogen phosphate, sodium chloride and water.

In some preferred embodiments of the present invention, the phosphate buffer comprises disodium hydrogen phosphate and potassium dihydrogen phosphate, and has a pH of 6.0 - 8.0 (in particular 6.5 - 7.5, e.g. 7.2) and an osmotic pressure of 300 - 700 mOsm/kg (in particular 600 mOsm/kg); more specifically, the phosphate buffer may be formed by combining disodium hydrogen phosphate (Na₂HPO₄), potassium dihydrogen phosphate (KH₂PO₄) and an aqueous solution of potassium chloride.

In some preferred embodiments of the present invention, the phosphate buffer comprises dipotassium hydrogen phosphate and potassium dihydrogen phosphate, and has a pH of 6.0 - 8.0 (in particular 6.5 - 7.5, e.g. 7.2) and an osmotic pressure of 500 - 650 mOsm/kg; more specifically, the phosphate buffer consists of dipotassium hydrogen phosphate, potassium dihydrogen phosphate, potassium chloride and water.

Specifically, the PCL microspheres have an average particle size range of 25 - 60 µm (e.g. 25, 30, 35, 40, 45, 50, 55, 60 µm).

Specifically, microspheres with a particle size of 25 - 50 µm make up ≥ 65% of the PCL microspheres.

Specifically, the PCL microspheres have a weight average molecular weight of 8000 - 80000 Da (e.g. 10000 Da, 20000 Da, 30000 Da, 40000 Da, 45000 Da, 60000 Da, 80000 Da), in particular 10000 - 60000 Da.

Specifically, in the polycaprolactone microsphere pre-dispersed composition, the content of the PCL microspheres may be 10 - 50 wt% (e.g. 10 wt%, 15 wt%, 20 wt%, 25 wt%, 30 wt%, 35 wt%, 40 wt%, 45 wt%, 50 wt%).

Specifically, the PCL microspheres may be prepared by the following method:
(1) aqueous phase formulation: a solution containing one or more of a film-forming agent, a surfactant and a thickener is used for mixing to serve as an aqueous phase;
(2) oil phase formulation: dry PCL is dissolved in an organic solvent;
(3) preparation of microspheres;
(4) volatilization of organic solvent in an emulsion, microsphere curing and filtration, washing, collection and drying.

Specifically, the film-forming agents include but are not limited to: polyvinyl alcohol, poly(propylene glycol), gelatin, gum Arabic, dextran sulfate, hyaluronic acid, pectin, carrageenan, etc.

Specifically, the surfactants include but are not limited to Tween, Span, sodium dodecylsulfate, sodium dodecylbenzenesulfonate, etc.

Specifically, the thickeners include but are not limited to: gelatin, starch, carrageenan, sodium alginate, chitosan, fructose, sodium carboxymethylcellulose, high-substituted carboxypropylcellulose, low-substituted carboxypropylcellulose, microcrystalline cellulose, carboxypropylmethylcellulose, carboxyethylcellulose, etc.

Specifically, the organic solvent is selected from: benzyl alcohol, dichloromethane, chloroform, chloroethane, dichloroethane, trichloroethane, ethyl acetate, ethyl formate, diethyl ether, cyclohexane or mixed solvents thereof.

Specifically, the preparation of microspheres in step (3) may employ a known method in the prior art such as membrane emulsification, spray drying, etc. For example, in the preparation of microspheres by membrane emulsification: using a pressure of 800 - 10000 mpa, preferably 800 - 3500 mpa and more preferably 1000 - 2000 mpa, an oil phase is forced through a membrane tube, with membrane pores 5 - 100 µm in size, into a continuous aqueous phase which has a flow rate of 50 - 10000 ml/min; the D50 of the microspheres thus prepared may be from 1 to 100 µm; the span is ≤ 2.0; the density, composition and form are uniform; the appearance is spherical, and the surface is smooth.

In some embodiments of the present invention, the PCL microspheres are prepared by the following method: an aqueous phase is formulated, an oil phase is formulated from polycaprolactone using dichloromethane, and after the aqueous phase and the oil continuous phase undergo emulsification, vacuum drying is performed to obtain polycaprolactone microspheres; the aqueous phase comprises one or more of polyvinyl alcohol, carboxymethylcellulose, sodium carboxymethylcellulose, Tween 80, gelatin, carboxypropylcellulose starch and sodium dodecylsulfate.

Specifically, the PCL microspheres may be sterile.

In a second aspect of the present invention, a method for preparing a polycaprolactone microsphere pre-dispersed composition is provided, comprising a step of (uniformly) dispersing dry polycaprolactone microspheres into a dispersion liquid, wherein the dispersion liquid is a phosphate buffer. Dispersion liquids for polycaprolactone microspheres are generally purified water, distilled water or normal saline, etc. The inventors found through research that the use of a phosphate buffer as a dispersion liquid helps to maintain optimal dispersion of the polycaprolactone microspheres and reduce the formation of bubbles.

Specifically, the polycaprolactone microspheres and the phosphate buffer have the definitions stated in the first aspect of the present invention.

In a third aspect of the present invention, a polycaprolactone injectable gel is provided, prepared by a method comprising a step of mixing the pre-dispersed composition described in the first aspect with a gel matrix, the gel matrix comprising (sodium) carboxymethylcellulose (i.e. carboxymethylcellulose or sodium carboxymethylcellulose) and a dispersion liquid.

Specifically, the polycaprolactone injectable gel is prepared by a method comprising adding the pre-dispersed composition described in the first aspect to a gel matrix and mixing.

Specifically, the dispersion liquid of the gel matrix is selected from: water (e.g. distilled water, purified water, water for injection), normal saline and a phosphate buffer.

In a preferred embodiment of the present invention, the dispersion liquid of the gel matrix is a phosphate buffer, in particular the phosphate buffer described in the first aspect of the present invention.

Specifically, a high content of polycaprolactone microspheres in the polycaprolactone injectable gel means that the content of polycaprolactone microspheres is higher than 20%, preferably higher than 25%, and more preferably higher than 30%.

Specifically, in the polycaprolactone injectable gel, the content of polycaprolactone microspheres is 28 - 38 wt% (e.g. 28%, 30%, 32%, 33%, 34%, 36%, 38%), in particular 30 - 36%.

Specifically, in the polycaprolactone injectable gel, the content of (sodium) carboxymethylcellulose is 1 - 5 wt% (e.g. 2 wt%, 2.3 wt%, 2.6 wt%, 2.9 wt%, 3 wt%, 4 wt%).

Specifically, the polycaprolactone injectable gel may further comprise a pH regulator, e.g. one or more of hydrochloric acid, sodium hydroxide and potassium hydroxide, etc.

Specifically, the polycaprolactone injectable gel has a pH of 6.0 - 8.0 (e.g. 6.0, 6.2, 6.4, 6.5, 6.6, 6.8, 7.0, 7.2, 7.4, 7.5, 7.6, 7.8, 8.0), in particular 6.5 - 8.0, preferably 7.0 - 7.5.

Specifically, the polycaprolactone injectable gel may further comprise an osmotic pressure regulator, e.g. one or more of potassium chloride, sodium chloride and glycerol, etc.

Preferably, the polycaprolactone injectable gel has an osmotic pressure of 300 - 600 mOsm/kg (e.g. 300, 350, 400, 450, 500, 550, 600 mOsm/kg), in particular 500 - 650 mOsm/kg, preferably 550 - 650 mOsm/kg.

In particular, the polycaprolactone injectable gel may further comprise an anaesthetic, e.g. lidocaine, tetracaine, etc.

Specifically, the polycaprolactone injectable gel may further comprise a lubricant, e.g. glycerol.

Specifically, the polycaprolactone injectable gel may further comprise an antimicrobial agent for improving an antimicrobial effect, so that the polycaprolactone injectable gel has a better antimicrobial effect during use, storage and transportation.

Specifically, the polycaprolactone injectable gel may further comprise an antiinflammatory agent, for reducing an inflammatory response of an organism.

Specifically, the polycaprolactone injectable gel may further comprise an antioxidant, for improving resistance to oxidation.

In a fourth aspect of the present invention, a method for preparing a polycaprolactone injectable gel is provided, comprising the following steps:
(1) (uniformly) dispersing polycaprolactone microspheres into a dispersion liquid I, to obtain a polycaprolactone pre-dispersed composition;
(2) dispersing (sodium) carboxymethylcellulose into a dispersion liquid II, to obtain a gel matrix;
(3) adding the polycaprolactone pre-dispersed composition to the gel matrix, and mixing (evenly).

Specifically, the dispersion I is a phosphate buffer, as stated in the first aspect of the present invention.

Specifically, the dispersion liquid II is selected from: water (e.g. distilled water, purified water, water for injection), normal saline and a phosphate buffer, in particular the phosphate buffer described in the first aspect of the present invention.

Specifically, the dispersion liquid I and dispersion liquid II may be the same phosphate buffer. For example, in some preferred embodiments of the present invention, the phosphate buffer comprises disodium hydrogen phosphate and sodium dihydrogen phosphate, and has a pH of 6.0 - 8.0 (in particular 6.5 - 7.5, e.g. 7.2) and an osmotic pressure of 500 - 650 mOsm/kg; more specifically, the phosphate buffer consists of disodium hydrogen phosphate, sodium dihydrogen phosphate, sodium chloride and water; in other preferred embodiments of the present invention, the phosphate buffer comprises disodium hydrogen phosphate and potassium dihydrogen phosphate, and has a pH of 6.0 - 8.0 (in particular 6.5 - 7.5, e.g. 7.2) and an osmotic pressure of 300 - 700 mOsm/kg (in particular 600 mOsm/kg); specifically, the phosphate buffer consists of disodium hydrogen phosphate, potassium dihydrogen phosphate, potassium chloride and water; in other preferred embodiments of the present invention, the phosphate buffer comprises dipotassium hydrogen phosphate and potassium dihydrogen phosphate, and has a pH of 6.0 - 8.0 (in particular 6.5 - 7.5, e.g. 7.2) and an osmotic pressure of 500 - 650 mOsm/kg; more specifically, the phosphate buffer consists of dipotassium hydrogen phosphate, potassium dihydrogen phosphate, potassium chloride and water.

Specifically, step (1) may further comprise a step of regulating pH.

Specifically, step (1) may further comprise a step of regulating osmotic pressure.

Specifically, step (1) may further comprise a step of sterilization.

Specifically, step (2) may further comprise a step of regulating pH.

Specifically, step (2) may further comprise a step of regulating osmotic pressure.

Specifically, step (2) may further comprise a step of sterilization.

Specifically, the mixing in step (3) is performed by stirring; the stirring speed may be 800 - 1200 r/min (e.g. 1000 r/min), and the stirring time may be 1 - 10 minutes (e.g. 5 minutes).

Specifically, step (3) further comprises a step of removing bubbles, e.g. performing stirring bubble removal treatment using a vacuum stirring bubble removal machine.

In a fifth aspect of the present invention, provided is the use of the polycaprolactone pre-dispersed composition described in the first aspect for preparing a polycaprolactone injectable gel, an implant or a drug carrier.

In an embodiment of the present invention, the implant is a beauty implant, e.g. a subcutaneous implant (e.g. implanted into the forehead, the nasolabial folds, the middle of the face, the nose, the lower jaw and the hands, etc. to reduce wrinkles, folds, scars, aging, etc.); in some embodiments of the present invention, the implant is a skin filler.

In another embodiment of the present invention, the implant is an implant for treating a disease, e.g. a stent.

The polycaprolactone microsphere pre-dispersed composition of the present invention has the following beneficial effects:
The PCL microspheres can continue to retain an intact spherical form, with a smooth surface and good dispersion, and a reduced rate of degradation, and bubbles can be reduced during mixing, helping to ensure stable product quality, and reducing the probability of nodules and granulomas forming due to non-uniform dispersion of the microspheres after injection into the body. The polycaprolactone injectable gel prepared therefrom has better application value.

### Brief Description of the Drawings

Fig. 1 shows a micrograph of undispersed, dry polycaprolactone microspheres.
Fig. 2 shows a micrograph of polycaprolactone microspheres after dispersion in distilled water.
Fig. 3 shows an electron micrograph of a polycaprolactone microsphere after dispersion in distilled water.
Fig. 4 shows a micrograph of polycaprolactone microspheres after dispersion in 0.9% normal saline.
Fig. 5 shows an electron micrograph of polycaprolactone microspheres after dispersion in 0.9% normal saline.
Fig. 6 shows a micrograph of polycaprolactone microspheres after dispersion in isosmotic phosphate buffer 3-2 of the sodium system.
Fig. 7 shows an electron micrograph of polycaprolactone microspheres after dispersion in isosmotic phosphate buffer 3-2 of the sodium system.
Fig. 8 shows a micrograph of polycaprolactone microspheres after dispersion in isosmotic phosphate buffer 3-5 of potassium system 1.
Fig. 9 shows an electron micrograph of polycaprolactone microspheres after dispersion in isosmotic phosphate buffer 3-5 of potassium system 1.
Fig. 10 shows a micrograph of polycaprolactone microspheres after dispersion in isosmotic phosphate buffer 3-8 of potassium system 2.
Fig. 11 shows an electron micrograph of polycaprolactone microspheres after dispersion in isosmotic phosphate buffer 3-8 of potassium system 2.
Fig. 12 shows a micrograph of polycaprolactone microspheres after dispersion in buffer (4-1-6) with osmotic pressure 600 mOsm/kg of the sodium system.
Fig. 13 shows a micrograph of polycaprolactone microspheres after dispersion in buffer (4-2-6) with osmotic pressure 600 mOsm/kg of potassium system 1.
Fig. 14 shows a micrograph of polycaprolactone microspheres after dispersion in buffer (4-3-6) with osmotic pressure 600 mOsm/kg of potassium system 2.
Fig. 15 shows an electron micrograph of polycaprolactone microspheres after dispersion in buffer (4-2-6) with osmotic pressure 600 mOsm/kg of potassium system 1.
Fig. 16 shows a photograph of the appearance of the injectable gel containing polycaprolactone microspheres of Example 11.
Fig. 17 shows a micrograph of the injectable gel containing polycaprolactone microspheres of Example 11.
Fig. 18 shows a photograph of the appearance of the injectable gel containing polycaprolactone microspheres of Example 12 after stirring at 1000 r/min.
Fig. 19 shows a photograph of the appearance of the injectable gel containing polycaprolactone microspheres of Example 12 after stirring at 2200 r/min.
Fig. 20 shows a micrograph of the injectable gel containing polycaprolactone microspheres of Example 12 after stirring at 2200 r/min.
Fig. 21 shows a photograph of the appearance of the injectable gel containing polycaprolactone microspheres of Example 13 after stirring at 1000 r/min.
Fig. 22 shows a photograph of the appearance of the injectable gel containing polycaprolactone microspheres of Example 13 after stirring at 2200 r/min.

### Particular embodiments

Unless otherwise defined, all scientific and technical terms used in the present invention have the same meanings as commonly understood by those skilled in the art.

The content disclosed in the various publications, patents and disclosed patent specifications cited herein are incorporated herein in their entirety by reference.

The technical solution of the present invention is described clearly and completely below in conjunction with embodiments of the present invention; obviously, the embodiments described are merely some, not all of the embodiments of the present invention. All other embodiments obtained by those skilled in the art without inventive effort on the basis of the embodiments in the present invention are included in the scope of protection thereof.

The measurement methods used in the embodiments below have the following specific operations:

### Measurement of morphology and particle size of dry microspheres

A suitable amount of dried microspheres is taken, approximately 0.5 cm of electrically conductive adhesive tape is cut and stuck onto a sample tray, a small amount of powder is scraped up and stuck onto the electrically conductive adhesive tape, a bulb syringe is used to blow excess floating powder off the electrically conductive adhesive tape, sputter coating with gold is carried out, a sample platform is placed into an instrument to perform observation, and a thermal field emission scanning electron microscope (e.g. JEOL- JSM-7001F) is used to capture an electron micrograph.

A small amount of dried microspheres is taken and dispersed in dispersion liquid, and stirred at high speed or subjected to ultrasound for 10 min; once the microspheres are fully dispersed, dropwise addition to a quartz dish is performed, and a Zeta particle size analyser is used to determine the particle size distribution of the microspheres.

### Measurement of morphology and particle size of dispersed microspheres

A small amount of dried microspheres is taken and dispersed in dispersion liquid, stirred at high speed or subjected to ultrasound for 10 min, and a microscope (XP-550C polarizing microscope) is used to observe microsphere dispersion and bubble formation.

A small amount of dried microspheres is taken and dispersed in dispersion liquid, and stirred at high speed or subjected to ultrasound for 10 min; once the microspheres are fully dispersed, dropwise addition to a quartz dish is performed, and a Zeta particle size analyser is used to determine the particle size distribution of the microspheres.

### Measurement of degradation of microspheres in dispersion liquid

6 portions of PCL microspheres of the same mass are separately weighed out (W0), then separately immersed in distilled water dispersion liquid, and placed in a shaking table at 37°C, with a shaking speed of 60 r/min. The dispersion liquid is changed each week, and measurement is carried out for 48 weeks, with 8 weeks as one degradation period. At the end of each degradation period, a sample is taken out, rinsed thoroughly in distilled water, and freeze-dried under vacuum until the weight is constant (Wₜ). The degradation rate = (W₀-Wₜ)/W₀×100% is calculated.

### Measurement of degradation of microspheres in gel

6 portions of PCL microspheres of the same mass are separately weighed out (W₀), then separately mixed into distilled water as dispersion liquid, and corresponding carboxymethylcellulose (or sodium carboxymethylcellulose) gels are prepared, then placed in a shaking table at 37°C, with a shaking speed of 60 r/min. Measurement is carried out for 48 weeks, with 8 weeks as one degradation period. At the end of each degradation period, 1 sample is taken out, microspheres are collected by centrifugation and rinsed thoroughly in distilled water, and freeze-dried under vacuum until the weight is constant (Wt), so as to obtain the degradation rate (W₀-Wₜ)/W₀×100% of PCL microspheres in the gel prepared from the dispersion liquid.

For the measurement of degradation rate in carboxymethylcellulose (or sodium carboxymethylcellulose) gels prepared with 0.9% normal saline and PBS as dispersion liquids, the preparation and measurement process is the same as that described above in which distilled water serves as the dispersion liquid.

### 1. Preparation of dry polycaprolactone microspheres:

### Example 1

10 to 20 grams of PCL with a Mw of 8 kDa are dissolved in dichloromethane (DCM, 10 - 20 w/w%), and the solution is dispersed in 1000 ml of water containing 0.1% - 5% MC; using a solvent extraction method, stirring is performed (1000 rpm), and the microspheres obtained are filtered, washed and dried. Particles of average diameter 40 µm are obtained; microspheres of particle size 25 - 50 µm make up 68.6% of the PCL microspheres, with Span 1.18 and a yield of about 67%.

### Example 2

53.9 g of PCL with a Mw of 10000 Da are weighed out and dissolved in dichloromethane (DCM, viscosity 700 cp); using a membrane emulsification method, with a pressure of 800 mpa, the oil phase solution is dispersed into an aqueous solution which is sheared at high speed and contains (1%) polyvinyl alcohol, (0.1%) Tween 80 aqueous solution and (2.2%) sodium carboxymethylcellulose; and the microspheres obtained are filtered, washed and dried. The microspheres have an average diameter of about 50 µm; microspheres of particle size 25 - 50 µm make up 70.4% of the PCL microspheres, with Span 1.19 and a yield of about 70%.

### Example 3

54.9 g of PCL with a Mw of 40000 Da are weighed out and dissolved in dichloromethane (DCM, viscosity 800 cp); using a high-speed homogenization emulsification method, the oil phase solution is dispersed into an aqueous solution which is sheared at high speed and contains (1%) polyvinyl alcohol, (0.5%) gelatin aqueous solution and (3.7%) sodium carboxymethylcellulose; and the microspheres obtained are filtered, washed and dried. The microspheres have an average diameter of about 55 µm; microspheres of particle size 25 - 50 µm make up 65.6% of the PCL microspheres, with Span 1.19 and a yield of about 65%.

### Example 4

54.9 g of PCL with a Mw of 60000 Da are weighed out and dissolved in dichloromethane (DCM, viscosity 800 cp); using a membrane emulsification method, with a pressure of 800 mpa, the oil phase solution is dispersed into an aqueous solution which is sheared at high speed and contains (1%) polyvinyl alcohol and (2.2%) carboxypropylcellulose; and the microspheres obtained are filtered, washed and dried. The microspheres have an average diameter of about 60 µm; microspheres of particle size 25 - 50 µm make up 66.2% of the PCL microspheres, with Span 1.19 and a yield of about 55%.

### Example 5

53.9 g of PCL with a Mw of 80000 Da are weighed out and dissolved in dichloromethane (DCM, viscosity 700 cp); using a membrane emulsification method, with a pressure of 800 mpa, the oil phase solution is dispersed into an aqueous solution which is sheared at high speed and contains (1%) polyvinyl alcohol, (0.1%) sodium dodecylsulfate solution and (2.2%) sodium carboxymethylcellulose; and the microspheres obtained are filtered, washed and dried. The microspheres have an average diameter of about 50 µm; microspheres of particle size 25 - 50 µm make up 73.3% of the PCL microspheres, with Span 1.19 and a yield of about 70%.

### 2. Dispersion of dry polycaprolactone microspheres:

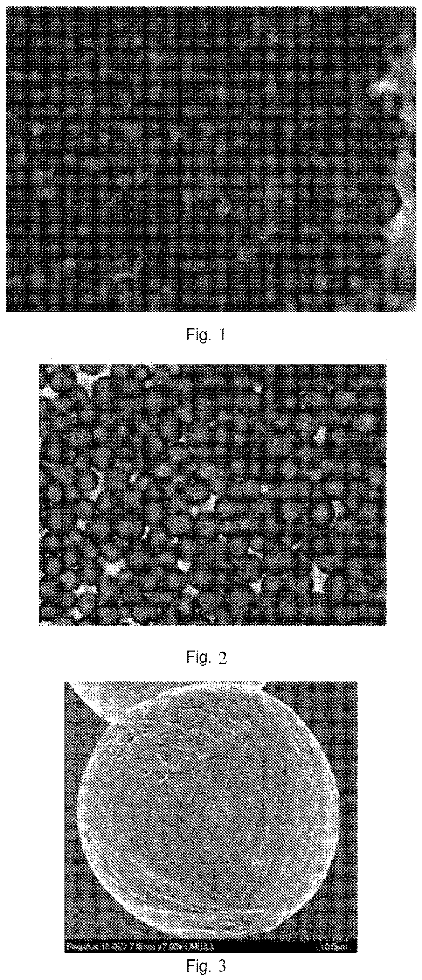

In each of the Examples below, the PCL microspheres made by the method in Example 2 are used to investigate the effect of different dispersion liquids on the degree of dispersion, bubble content and smoothness of the PCL microspheres obtained. Fig. 1 shows a micrograph (XP-550C polarizing microscope, 10x) of undispersed microspheres, which exhibit agglomeration.

### Example 6: dispersion liquid 1 (distilled water)

The microspheres obtained are dispersed in distilled water, and a microscope and an electron microscope are used to observe the surface smoothness, the completeness of spherical form, and the degree of dispersion of the PCL. Figs. 2 and 3 respectively show a micrograph (XP-550C polarizing microscope, 10x) and an electron micrograph of the PCL microspheres.

As shown in Fig. 2, the microspheres are not dispersed very well in distilled water.

As shown in Fig. 3, the surface smoothness of the microspheres is reduced, and the completeness of their spherical form is impaired.

### Example 7: dispersion liquid 2 (0.9% normal saline)

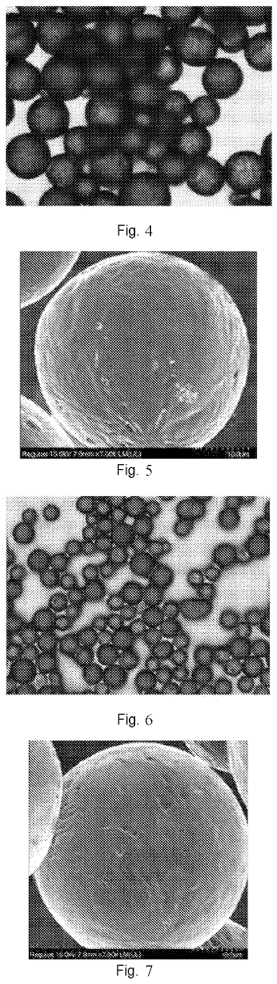

The microspheres obtained are dispersed in 0.9% normal saline, and a microscope and an electron microscope are used to observe the surface smoothness, the completeness of spherical form, and the degree of dispersion of the PCL. Figs. 4 and 5 respectively show a micrograph (XP-550C polarizing microscope, 20x) and an electron micrograph of the PCL microspheres.

As shown in Fig. 4, compared with distilled water, the degree of dispersion of microspheres in normal saline is improved; and as can be seen from the electron micrograph in Fig. 5, the surface smoothness of the microspheres is impaired to a lesser extent, and the completeness of spherical form is improved.

### Example 8: dispersion liquid 3: (isosmotic phosphate buffers with different pH values)

### 1. Preparation of isosmotic phosphate buffers of sodium system

Disodium hydrogen phosphate: 0.1M disodium hydrogen phosphate (Na₂HPO₄), stored at 4°C;
sodium dihydrogen phosphate: 0.1M sodium dihydrogen phosphate (NaH₂PO₄^{•}H₂O).

Solutions of the required pH are formulated according to the following table:

**Table 1: Dispersion liquid formulas**

| Dispersion liquid no. | Na₂HPO₄/ml | NaH₂PO₄/ml | pH | Amount of NaCl added to maintain isosmoticity (g) |
|---|---|---|---|---|
| 3-1 | 50 | 50 | 6 | 0.17 |
| 3-2 | 80 | 20 | 7.2 | 0.054 |
| 3-3 | 90 | 10 | 8 | 0.015 |

The microspheres obtained are dispersed into the abovementioned dispersion liquids 3-1 to 3-3, and a microscope and an electron microscope are used to observe the surface smoothness (smooth +++++; nearly smooth ++++; a few uneven points +++; quite a large number of uneven points ++; a large number of uneven points +), the completeness of spherical form (spherical +++++; nearly spherical ++++; 1 - 2 ellipsoid vertices +++; 2 - 4 ellipsoid vertices ++; 5 or more ellipsoid vertices +), and the degree of dispersion (no clustering +++++; nearly no clustering ++++; slight clustering +++; quite a large number of clusters of 3 or more microspheres ++; a large number of clusters of 3 or more microspheres+) of the PCL. The observation results are shown in the table below; Figs. 6 and 7 respectively show a micrograph (XP-550C polarizing microscope, 10x) and an electron micrograph of the PCL microspheres in dispersion liquid 3-2.

**Table 2: Dispersion liquid investigation results**

| Dispersion liquid no. | Surface smoothness | Completeness of spherical form | Degree of dispersion |
|---|---|---|---|
| 3-1 | +++ | +++ | +++ |
| 3-2 | +++ | +++ | +++ |
| 3-3 | +++ | +++ | +++ |

It can be seen from Figs. 6 - 7 (corresponding to dispersion liquid 3-2) and the table above that the degree of dispersion of microspheres in the isosmotic phosphate buffer of the sodium system is further improved, and it can be seen from the electron micrograph that the surface smoothness of the microspheres is impaired to a lesser extent, and the completeness of spherical form is improved.

### 2. Preparation of isosmotic phosphate buffers of potassium system 1:

Disodium hydrogen phosphate: 0.1M disodium hydrogen phosphate (Na₂HPO₄), stored at 4°C;
potassium dihydrogen phosphate: 0.1M potassium dihydrogen phosphate (KH₂PO₄), stored at 4°C.

Solutions of the required pH are formulated according to the following table:

**Table 3: Dispersion liquid formulas**

| Dispersion liquid no. | Na₂HPO₄/ml | KH₂PO₄/ml | pH | Amount of KCl added to maintain isosmoticity (g) |
|---|---|---|---|---|
| 3-4 | 40 | 60 | 6 | 0.252 |
| 3-5 | 70 | 30 | 7.2 | 0.111 |
| 3-6 | 95 | 5 | 8 | 0 |

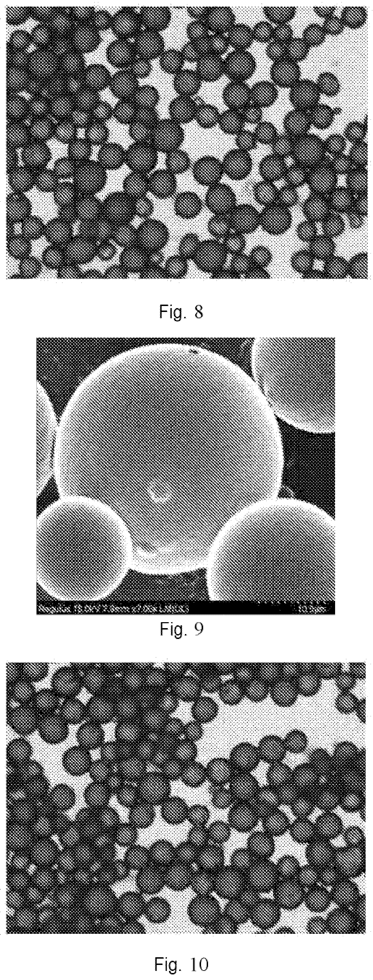

The microspheres obtained are dispersed in the abovementioned dispersion liquids 3-4 to 3-6, and a microscope and an electron microscope are used to observe the surface smoothness, the completeness of spherical form, and the degree of dispersion of the PCL. The observation results are shown in the table below; Figs. 8 and 9 respectively show a micrograph (XP-550C polarizing microscope, 10x) and an electron micrograph of the PCL microspheres in dispersion liquid 3-5.

**Table 4: Dispersion liquid investigation results**

| Dispersion liquid no. | Degree of dispersion of microspheres | Completeness of microspheres | Sphericity of microspheres |
|---|---|---|---|
| 3-4 | +++ | +++ | +++ |
| 3-5 | ++++ | ++++ | ++++ |
| 3-6 | +++ | +++ | +++ |

It can be seen from Figs. 8 - 9 (corresponding to dispersion liquid 3-5) and the table above that the degree of dispersion of microspheres in the isosmotic phosphate buffer of potassium system 1 is further improved, and it can be seen from the electron micrograph that the surface smoothness of the microspheres is impaired to a very small extent, and the completeness of spherical form is high.

### 3. Preparation of isosmotic phosphate buffers of potassium system 2:

Dipotassium hydrogen phosphate (bivalent): 0.1M dipotassium hydrogen phosphate (K₂HPO₄), stored at 4°C;
potassium dihydrogen phosphate: 0.1M potassium dihydrogen phosphate (KH₂PO₄), stored at 4°C.

Solutions of the required pH are formulated according to the following table:

**Table 5: Dispersion liquid formulas**

| Dispersion liquid no. | K₂HPO₄/ml | KH₂PO₄/ml | pH | Amount of KCl added to maintain isosmoticity (g) |
|---|---|---|---|---|
| 3-7 | 40 | 60 | 6 | 0.252 |
| 3-8 | 70 | 30 | 7.2 | 0.112 |
| 3-9 | 95 | 5 | 8 | 0 |

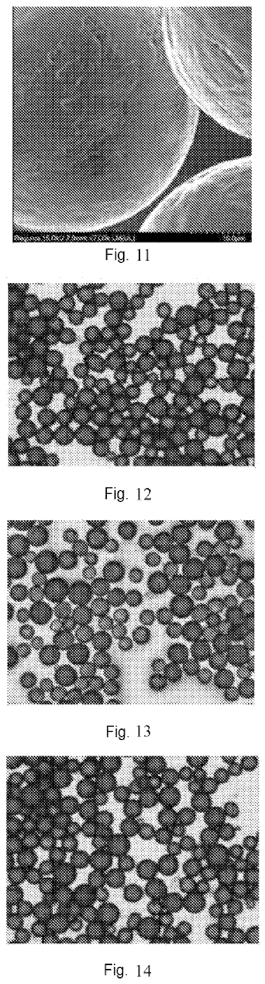

The microspheres obtained are dispersed in the abovementioned dispersion liquids 3-7 to 3-9, and a microscope and an electron microscope are used to observe the surface smoothness, the completeness of spherical form, and the degree of dispersion of the PCL. The observation results are shown in the table below; Figs. 10 and 11 respectively show a micrograph (XP-550C polarizing microscope, 10x) and an electron micrograph of the PCL microspheres in dispersion liquid 3-8.

**Table 6: Dispersion liquid investigation results**

| Dispersion liquid no. | Degree of dispersion of microspheres | Completeness of microspheres | Sphericity of microspheres |
|---|---|---|---|
| 3-7 | +++ | +++ | +++ |
| 3-8 | +++ | +++ | +++ |
| 3-9 | +++ | +++ | +++ |

It can be seen from Figs. 10 - 11 (corresponding to dispersion liquid 3 -8) and the table above that the microspheres are similar in the isosmotic phosphate buffer of potassium system 2 and in the sodium-system dispersion liquid, in both cases having a good degree of dispersion, and it can be seen from the electron micrograph that the surface smoothness of the microspheres is impaired to a very small extent, and the completeness of spherical form is high.

### Example 9: dispersion liquid 4: (phosphate buffers with different osmotic properties)

### 1. Preparation of buffers with different osmotic properties, based on isosmotic phosphate buffer of sodium system (pH 7.2, 100 ml)

Referring to dispersion liquid 3-2 in Table 2 of Example 8, the osmotic pressure is adjusted by adjusting the amount of NaCl added, to obtain dispersion liquids 4-1-1 to 4-1-8.

The microspheres obtained are dispersed in the abovementioned dispersion liquids 4-1-1 to 4-1-8, and a microscope and an electron microscope are used to observe the surface smoothness, the completeness of spherical form, and the degree of dispersion of the PCL. The observation results are shown in the table below.

**Table 7: Dispersion liquid investigation results**

| Dispersio n liquid no. | Amount of NaCl added to adjust osmotic pressure (g) | Osmotic pressure mOsm/kg | Degree of dispersion of microspheres | Completeness of microspheres | Sphericity of microspher es |
|---|---|---|---|---|---|
| 4-1-1 | 0.054 | 300 | +++ | +++ | +++ |
| 4-1-2 | 0.200 | 350 | +++ | +++ | +++ |
| 4-1-3 | 0.347 | 400 | +++ | +++ | +++ |
| 4-1-4 | 0.493 | 450 | +++ | +++ | +++ |
| 4-1-5 | 0.640 | 500 | +++ | +++ | +++ |
| 4-1-6 | 0.933 | 600 | ++++ | ++++ | ++++ |
| 4-1-7 | 1.080 | 650 | ++++ | +++ | +++ |
| 4-1-8 | 1.226 | 700 | +++ | +++ | +++ |

### 2. Preparation of buffers with different osmotic properties, based on isosmotic phosphate buffer of potassium system 1 (pH 7.2, 100 ml)

Referring to dispersion liquid 3-5 in Table 3 of Example 8, the osmotic pressure is adjusted by adjusting the amount of KCl added, to obtain dispersion liquids 4-2-1 to 4-2-8.

The microspheres obtained are dispersed in the abovementioned dispersion liquids 4-2-1 to 4-2-8, and a microscope and an electron microscope are used to observe the surface smoothness, the completeness of spherical form, and the degree of dispersion of the PCL. The observation results are shown in the table below.

**Table 8: Dispersion liquid investigation results**

| Dispersion liquid no. | Amount of KCl added to adjust osmotic pressure (g) | Osmotic pressure mOsm/kg | Degree of dispersion of microspheres | Completeness of microspheres | Sphericity of microspheres |
|---|---|---|---|---|---|
| 4-2-1 | 0.111 | 300 | ++++ | ++++ | ++++ |
| 4-2-2 | 0.299 | 350 | ++++ | ++++ | ++++ |
| 4-2-3 | 0.487 | 400 | ++++ | ++++ | ++++ |
| 4-2-4 | 0.675 | 450 | ++++ | ++++ | ++++ |
| 4-2-5 | 0.863 | 500 | ++++ | ++++ | ++++ |
| 4-2-6 | 1.239 | 600 | +++++ | +++++ | +++++ |
| 4-2-7 | 1.427 | 650 | ++++ | ++++ | ++++ |
| 4-2-8 | 1.614 | 700 | +++ | +++ | +++ |

### 3. Preparation of buffers with different osmotic properties, based on isosmotic phosphate buffer of potassium system 2 (pH 7.2, 100 ml)

Referring to dispersion liquid 3-8 in Table 5 of Example 8, the osmotic pressure is adjusted by adjusting the amount of KCl added, to obtain dispersion liquids 4-3-1 to 4-3-8.

The microspheres obtained are dispersed in the abovementioned dispersion liquids 4-3-1 to 4-3-8, and a microscope (XP-550C polarizing microscope, 10x) and an electron microscope are used to observe the surface smoothness, the completeness of spherical form, and the degree of dispersion of the PCL. The observation results are shown in the table below.

**Table 9: Dispersion liquid investigation results**

| Dispersion liquid no. | Amount of KCl added to adjust osmotic pressure (g) | Osmotic pressure mOsm/kg | Degree of dispersion of microspheres | Completeness of microspheres | Sphericity of microspheres |
|---|---|---|---|---|---|
| 4-3-1 | 0.112 | 300 | +++ | +++ | +++ |
| 4-3-2 | 0.300 | 350 | +++ | +++ | +++ |
| 4-3-3 | 0.488 | 400 | +++ | +++ | +++ |
| 4-3-4 | 0.676 | 450 | +++ | +++ | +++ |
| 4-3-5 | 0.864 | 500 | +++ | +++ | +++ |
| 4-3-6 | 1.239 | 600 | ++++ | ++++ | ++++ |
| 4-3-7 | 1.428 | 650 | ++++ | +++ | +++ |
| 4-3-8 | 1.615 | 700 | +++ | +++ | +++ |

It can be seen from Figs. 12 - 14 (respectively corresponding to dispersion liquids 4-1-6, 4-2-6 and 4-3-6) and the tables above that, compared to dispersion liquids with other osmotic pressures in the sodium system, potassium system 2 and potassium system 1, when microspheres are dispersed in the buffer with osmotic pressure 600 in potassium system 1, they have a good degree of dispersion, the surface smoothness of the microspheres is impaired to an extremely small extent, and no effective change in the completeness of spherical form is observed.

### Example 10: Effect of dispersion liquid on degradation of microspheres

18 portions of 750 mg (W₀) of PCL microspheres (weight average molecular weight of PCL = 40000) are weighed out, then the 6 portions are separately immersed in a solution of 10 mL of PBS (the dispersion liquid 4-2-6 in Example 9), 10 mL of 0.9% normal saline and 10 mL of distilled water, and placed in a shaking table at 37°C, with a shaking speed of 60 r/min. The solution is changed each week, and measurement is carried out for 48 weeks, with 8 weeks as one degradation period. At the end of each degradation period, a sample is taken out, rinsed thoroughly in distilled water, and freeze-dried under vacuum until the weight is constant (Wt). Degradation rate =(W₀-Wₜ)/W₀×100%.

**Table 10: Degradation rate investigation results**

| Degradation period (weeks) | Weight | | | Degradation rate | | |
|---|---|---|---|---|---|---|
| Dispersion liquid | Distilled water | 0.9% normal saline | PBS | Distilled water | 0.9% normal saline | PBS |
| 0 | 750 | 750 | 750 | 0.00% | 0.00% | 0.00% |
| 8 | 727.5 | 731.25 | 740.625 | 3.00% | 2.50% | 1.25% |
| 16 | 708.75 | 718.125 | 731.25 | 5.50% | 4.25% | 2.50% |
| 24 | 678.75 | 684.375 | 703.125 | 9.50% | 8.75% | 6.25% |
| 32 | 485.625 | 500.625 | 590.625 | 35.25% | 33.25% | 21.25% |
| 40 | 438.75 | 453.75 | 543.75 | 41.50% | 39.50% | 27.50% |
| 48 | 397.5 | 420 | 515.625 | 47.00% | 44.00% | 31.25% |

It can be seen from the above table that the degradation rate of PCL that is pre-dispersed in PBS (the dispersion liquid 4-2-6 in Example 9) as dispersion liquid is lower than in the case of normal saline, and significantly lower than in the case of distilled water. It can thus be seen that PBS as a dispersion liquid can effectively reduce PCL degradation, and the retention of the smooth spherical form of the microspheres helps to reduce the rate of microsphere degradation.

### 1. Preparation of polycaprolactone microsphere gel:

### Example 11:

An injectable gel of polycaprolactone microspheres is prepared, the specific steps being as follows:
(1) Stir CMC with deionized water, and mix evenly to obtain a CMC suspension; add a sodium hydroxide solution to the CMC suspension, and mix evenly; then add hydrochloric acid to adjust the pH value to neutral, to obtain a CMC-Na gel, and measure out 33 g of the CMC-Na gel;
(2) formulate the dispersion liquid 4-2-6 in Example 9 with phosphate buffer;
(3) disperse 33 g of sterilized PCL microspheres in 33 g of dispersion liquid, the dispersion liquid being phosphate buffer with a pH value of about 7, with a stirring rotation speed of 500 r/min, to form a uniform pre-dispersed composition;
(4) add the pre-dispersed composition to the CMC-Na gel (the content of CMC-Na in the final product being 2.6 wt%), add an amount of glycerol equivalent to 1 wt% of the product, and use a glass rod or a fine scraper to perform preliminary stirring for 5 min;
(5) prepare a preliminarily mixed sample, and use a vacuum stirring bubble removal machine to perform stirring bubble removal treatment, for a duration of 6 min, at a rotation speed of 1000 r/min. Let the amount of PCL used in the final product be 33 wt%.

### Example 12: (comparative product 1): PCL microspheres are added to CMC-Na gel directly, without preparing a pre-dispersed composition

An injectable gel of polycaprolactone microspheres is prepared, the specific steps being as follows:
(1) Dissolve 2.6 g of CMC-Na powder in 63.4 g of the phosphate buffer prepared in Example 11, to form a gel;
(2) add 33 g of sterilized PCL microspheres to the CMC-Na gel (the content of CMC-Na in the final product being 2.6 wt%), add an amount of glycerol equivalent to 1 wt% of the product, and use a glass rod or a fine scraper to perform preliminary stirring for 5 min;
(3) prepare a preliminarily mixed sample, and use a vacuum stirring bubble removal machine to perform stirring bubble removal treatment, for a duration of 6 min, at a rotation speed of 1000 r/min. Let the amount of PCL used in the final product be 33 wt%. Thereafter, increase the rotation speed to 2200 r/min to treat for 10 min.

### Example 13: (comparative product 2): PCL microspheres, CMC-Na powder, phosphate buffer and glycerol are mixed directly, without preparing a pre-dispersed composition

An injectable gel of polycaprolactone microspheres is prepared, the specific steps being as follows:
(1) 33 g of PCL microspheres, 63.4 g of phosphate buffer prepared by the method of Example 11, and 1 g of glycerol are stirred at a rotation speed of 500 r/min, to form a uniform suspension;
(2) 2.6 g of CMC-Na powder are put in a vessel; to the vessel are added the suspension formed by the PCL microspheres, phosphate buffer and 1 g of glycerol, the content of CMC-NA in the product being 2.6 wt%, and a glass rod or a fine scraper is used to perform preliminary stirring for 5 min;
(3) a preliminarily mixed sample is prepared, and a vacuum stirring bubble removal machine is used to perform stirring bubble removal treatment, for a duration of 5 min, at a rotation speed of 1000 r/min. The amount of PCL used in the final product is set at 33 wt%; thereafter, the rotation speed is increased to 2200 r/min to treat for 10 min.

### Example 14: Comparison of gel product containing no microspheres with product in which PCL microspheres are added directly to CMC-Na gel

2.6 g of CMC-Na powder are dissolved in 96.4 g of phosphate buffer prepared in Example 11, and 1 wt% glycerol is added to form a gel (content of CMC-Na in the gel being 2.6 wt%).

The physicochemical properties of the abovementioned gel containing no microspheres and the CMC-Na gel containing microspheres which is prepared in Example 12 are compared, and the results are shown in the table below.

**Table 11: Shear viscosity comparison results**

| | Viscosity average molecular weight | Shear viscosity/ mPa•s | |
|---|---|---|---|
| Sample | CMC-Na | CMC-Na gel containing no microspheres | CMC-Na gel containing PCL microspheres |
| | 800000 - 1200000 | 53060 | 88946 |

It can be seen from the table above that after the addition of PCL microspheres to the CMC-Na gel, the shear viscosity of the polycaprolactone injectable gel formed is notably increased; at this time, the gel is more viscous, and is more difficult to mix evenly. In the prior art, mixing methods for high-viscosity fluids generally employ mixing with a high-speed shear stirrer as described above, or a high-viscosity homogenizer or other mixing method is employed; however, in this process, CMC gel will trap bubbles, and introduce additional heat into the system, causing shrinkage deformation of microspheres in the injectable gel, which will influence the injection result. For this reason, the method of adding the PCL microsphere pre-dispersed composition to the CMC gel is employed in Example 11 to reduce viscosity when the microspheres mix with the CMC-Na gel, reducing bubbles and increasing the mixing efficiency.

### Example 15: Comparison of bubbles and degree of microsphere dispersion in gel products in Examples 11 - 13

A comparison is performed of bubbles (no bubbles -; a few bubbles +; a small number of bubbles ++; quite a large number of bubbles +++; a large number of bubbles ++++; full of bubbles +++++) and degree of microsphere dispersion in the product prepared in Example 11 and the comparative products 1 - 2 prepared in Examples 12 - 13, and the results are shown in the table below.

**Table 12: Results for bubbles and degree of microsphere dispersion in gel products in Examples 11 - 13**

| | Example 11 | | Comparative product 1 | | Comparative product 2 | |
|---|---|---|---|---|---|---|
| | Phosphate buffer | | PCL added directly without the use of pre-dispersion | | Directly added to CMC-Na | |
| | Bubbles | Dispersion | Bubbles | Dispersion | Bubbles | Dispersion |
| Evaluation | - | ++++ | +++ | ++ | ++++ | + |
| Images | Figs. 16 - 17 | | Figs. 18 - 20 | | Figs. 21 - 22 | |

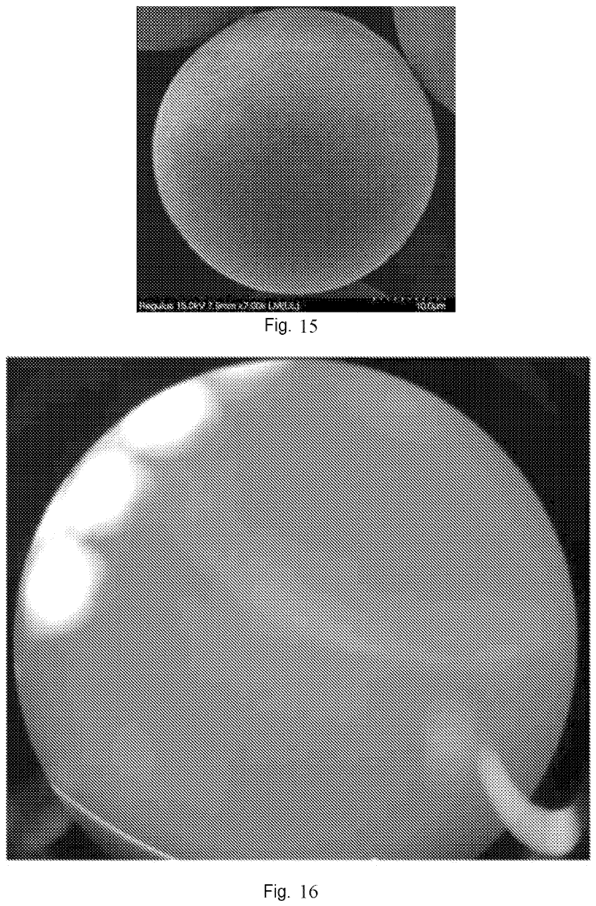

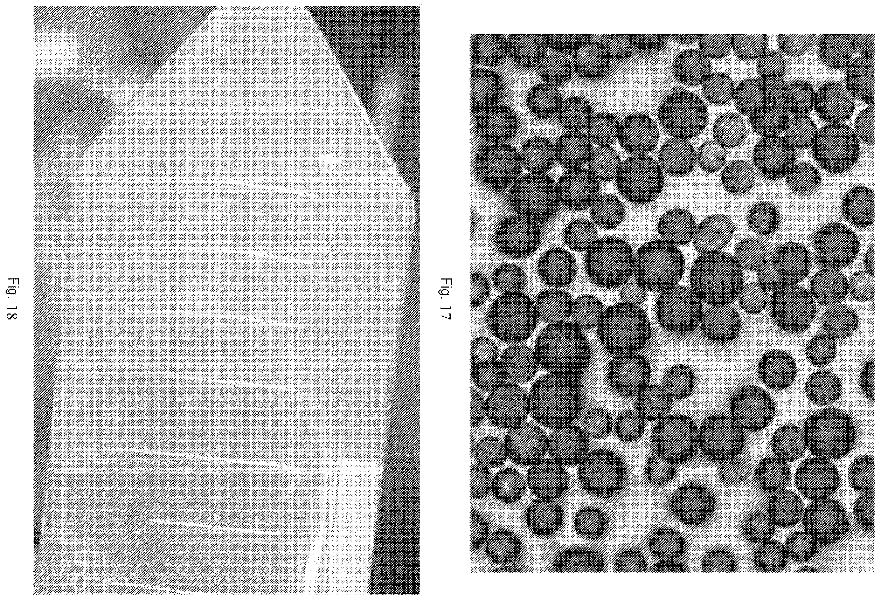

It can be seen from the micrograph (microscope BM2000, 10x) in Fig. 16 that, compared with the comparative products, when the pre-dispersed composition is used, the number of bubbles in the gel product is greatly reduced, and it can be seen from the micrograph (XP-550C polarizing microscope, 10x) in Fig. 17 that a gel product in which PCL microspheres are uniformly distributed can be obtained by employing a mixing rotation speed of 1000 r/min for 5 min, whereas the comparative products require the rotation speed and time to be increased for continued stirring.

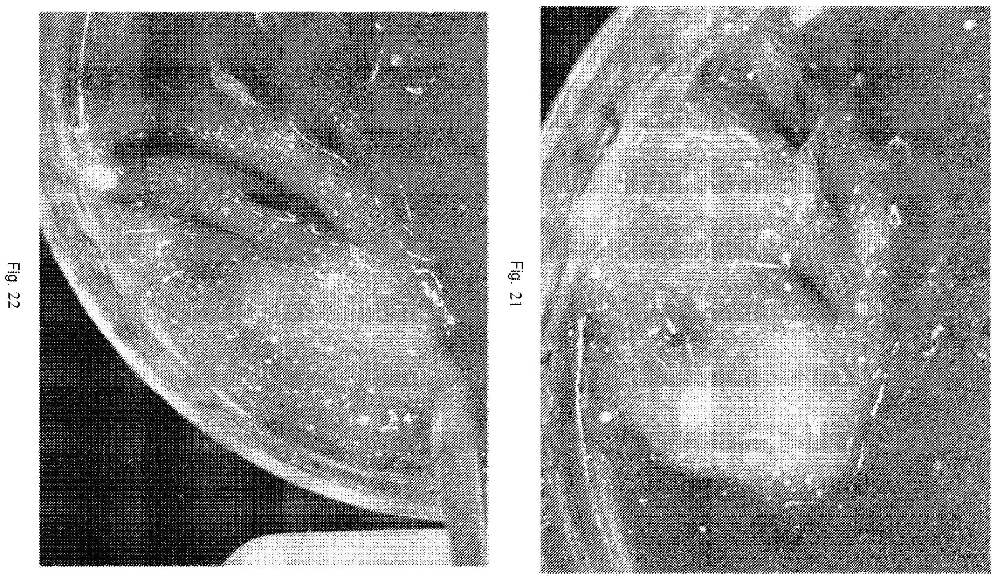

When no pre-dispersion step is employed, a large number of obvious bubbles are formed in both comparative products 1 and 2 during stirring (in Figs. 18 and 21, the large dark spheres which are whitish in the middle are bubbles); in comparative product 2 especially, when PCL microspheres, CMC-Na powder, phosphate buffer and glycerol are mixed directly, the number of bubbles is large. Furthermore, the CMC-Na forms a large number of white pieces due to inadequate mixing; stirring at a rotation speed of 1000 r/min for 5 min is insufficient, and it is relatively difficult to further mix the pieces of agglomerated CMC to obtain a uniform gel-like injection liquid.

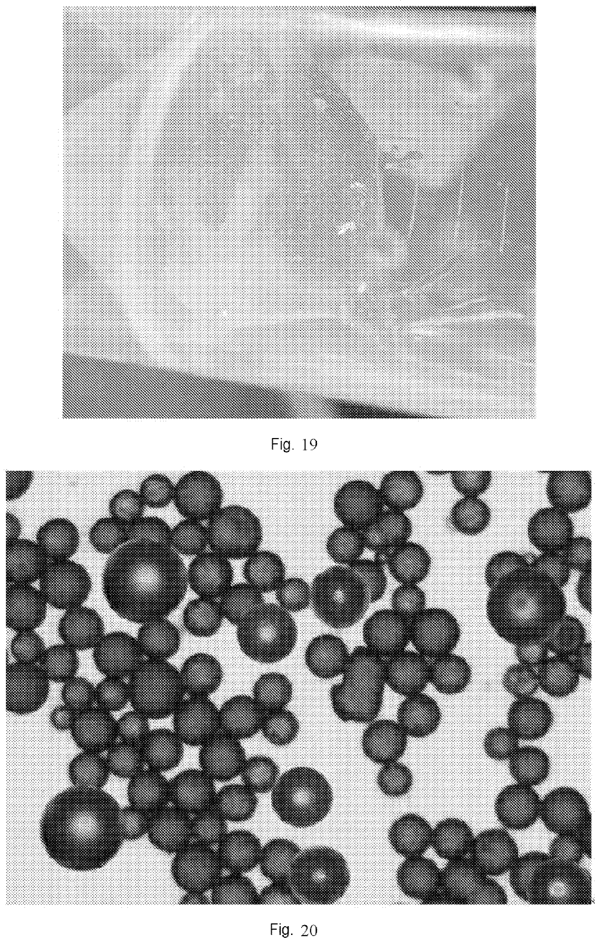

It can be seen from Fig. 20 of comparative product 1 (XP-550C polarizing microscope, 10x) that the gel contains a large number of bubbles and some of the microspheres are agglomerated, and the dispersion effect thereof is similar to that when deionized water is used as dispersion liquid; in both cases, there is a degree of clustering with incomplete dispersion.

A trial is conducted whereby a higher rotation speed is used, with a longer stirring time, for continued treatment of the gel. In comparative product 1, after increasing the rotation speed to 2200 r/min and treating for 10 min, as shown in Fig. 19, the uniformity of the gel is somewhat improved in comparison with before, with increased transparency, and large bubbles have been dispersed into small bubbles, but the number is still large. In comparative product 2, after increasing the rotation speed for the same treatment, a large number of bubbles are still present; although there is a slight reduction in agglomeration and clumping, the dispersion of the gel is still not uniform overall, and inferior to that of the re-treated sample in Example 12.

### Example 16: Effect of gel matrix on microsphere degradation

6 portions of 750mg (W₀) of PCL microspheres (the PCL having a weight average molecular weight of 40000 Da) are weighed out, then separately mixed into distilled water and then into sodium carboxymethylcellulose gel, then placed in a shaking table at 37°C, with a shaking speed of 60r/min. Measurement is carried out for 48 weeks, with 8 weeks as one degradation period. At the end of each degradation period, 1 sample is taken out, microspheres are collected by centrifugation and rinsed thoroughly in distilled water, and freeze-dried under vacuum until the weight is constant (Wt), so as to obtain the degradation rate of PCL microspheres in the gel prepared with distilled water as dispersion liquid.

For the degradation rate in sodium carboxymethylcellulose gels prepared with 0.9% normal saline and PBS (the osmotic pressure of which has been adjusted with KCl) of potassium system 1 (the dispersion liquid 4-2-6 in Example 9) as dispersion liquids, the preparation and measurement process is the same as that described above in which distilled water serves as the dispersion liquid.

**Table 14: Degradation rate investigation results**

| Degradation period (weeks) | Weight | | | Degradation rate | | |
|---|---|---|---|---|---|---|
| Dispersion liquid used | Distilled | 0.9% | PBS | Distilled | 0.9% | PBS |
| for CMC-Na gel | water | normal saline | | water | normal saline | |
| 0 | 750 | 750 | 750 | 0.00% | 0.00% | 0.00% |
| 8 | 731.4 | 735.9 | 745.95 | 2.48% | 1.88% | 0.54% |
| 16 | 712.65 | 722.775 | 736.575 | 4.98% | 3.63% | 1.79% |
| 24 | 682.65 | 689.025 | 708.45 | 8.98% | 8.13% | 5.54% |
| 32 | 489.525 | 505.275 | 595.95 | 34.73% | 32.63% | 20.54% |
| 40 | 442.65 | 458.4 | 549.075 | 40.98% | 38.88% | 26.79% |
| 48 | 401.4 | 424.65 | 520.95 | 46.48% | 43.38% | 30.54% |

It can be seen from the above table that the degradation rate of PCL in gel with PBS (the dispersion liquid 4-2-6 in Example 9) as dispersion liquid and prepared therefrom is lower than in the case of normal saline, and significantly lower than in the case of distilled water. It can thus be seen that PBS as a dispersion liquid can effectively reduce PCL degradation, and the retention of the smooth spherical form of the microspheres helps to reduce the rate of microsphere degradation.

The above research shows that the use of a phosphate buffer as a dispersion liquid, in particular a buffer made from disodium hydrogen phosphate and potassium dihydrogen phosphate with a pH of 6.0 - 8.0 and an osmotic pressure of 300 - 650 mOsm/kg, can maintain the smooth-surfaced spherical form of PCL microspheres very well, reducing or preventing the formation of bubbles; and further, the use of CMC-Na as a gel matrix, with KCl as an osmotic pressure regulator and an osmotic pressure of 300 - 650 mOsm/kg, can maintain the smooth-surfaced spherical form of PCL microspheres very well, and effectively reduce PCL microsphere degradation.

The above are merely preferred embodiments of the present patent, and it should be pointed out that without departing from the technical principles of the present patent, those skilled in the art could make improvements and substitutions, which should also be regarded as falling within the scope of protection of the present patent.

## Claims

1. A polycaprolactone microsphere pre-dispersed composition, comprising polycaprolactone microspheres and a dispersion liquid I, wherein the dispersion liquid I comprises a phosphate buffer, the phosphate buffer comprising a dibasic phosphate salt and/or a monobasic phosphate salt, and the dispersion liquid I having a pH of 7.0 - 7.5 and an osmotic pressure of 550 - 650 mOsm/kg;
the polycaprolactone microsphere pre-dispersed composition being made from dry polycaprolactone microspheres dispersed uniformly in dispersion liquid I.

2. The polycaprolactone microsphere pre-dispersed composition as claimed in claim 1, **characterized in that** the dibasic phosphate salt is selected from one or more of disodium hydrogen phosphate, dipotassium hydrogen phosphate or diammonium hydrogen phosphate, and the monobasic phosphate salt is selected from one or more of sodium dihydrogen phosphate, potassium dihydrogen phosphate or ammonium dihydrogen phosphate.

3. The polycaprolactone microsphere pre-dispersed composition as claimed in claim 2, **characterized in that** the dispersion liquid I further comprises a pH regulator.

4. The polycaprolactone microsphere pre-dispersed composition as claimed in claim 3, **characterized in that** the pH regulator is selected from one or more of hydrochloric acid, sodium hydroxide and potassium hydroxide.

5. The polycaprolactone microsphere pre-dispersed composition as claimed in claim 4, **characterized in that** the dispersion liquid I further comprises an osmotic pressure regulator.

6. The polycaprolactone microsphere pre-dispersed composition as claimed in claim 5, **characterized in that** the osmotic pressure regulator is selected from one or more of potassium chloride, sodium chloride and glycerol.

7. The polycaprolactone microsphere pre-dispersed composition as claimed in claim 6, wherein the dibasic phosphate salt is disodium hydrogen phosphate; the monobasic phosphate salt is potassium dihydrogen phosphate; the osmotic pressure is changed to 300 - 650 mOsm/kg; and the osmotic pressure regulator is potassium chloride.

8. The polycaprolactone microsphere pre-dispersed composition as claimed in any one of claims 1 - 7, **characterized in that** the polycaprolactone microspheres have an average particle size range of 25 - 60 µm, microspheres with a particle size of 25 - 50 µm make up ≥ 65% of the polycaprolactone microspheres, and the polycaprolactone microspheres have a weight average molecular weight of 8000 - 80000 Da.

9. A polycaprolactone injectable gel, comprising the polycaprolactone microsphere pre-dispersed composition as claimed in any one of claims 1 - 8 and a gel matrix, the gel matrix comprising a dispersion liquid II, and carboxymethylcellulose or sodium carboxymethylcellulose;
the dispersion liquid II of the gel matrix is selected from: water, normal saline or a phosphate buffer;
the polycaprolactone injectable gel has a pH of 7.0 - 7.5, and an osmotic pressure of 550 - 650 mOsm/kg.

10. The polycaprolactone injectable gel as claimed in claim 9, **characterized in that** in the polycaprolactone injectable gel, the content of polycaprolactone microspheres is 28 - 38 wt%, and the content of carboxymethylcellulose or sodium carboxymethylcellulose is 1 - 5 wt%.

11. A method for preparing the polycaprolactone injectable gel as claimed in claim 9 or 10, the method comprising the following steps:
(1) dispersing polycaprolactone microspheres into a dispersion liquid I, to obtain a polycaprolactone pre-dispersed composition;
(2) dispersing carboxymethylcellulose or sodium carboxymethylcellulose into a dispersion liquid II, to obtain a gel matrix;
(3) adding the polycaprolactone pre-dispersed composition of step (1) to the gel matrix of step (2), and mixing.

12. The use of the polycaprolactone microsphere pre-dispersed composition as claimed in any one of claims 1 - 8 for preparing a polycaprolactone injectable gel, an implant or a drug carrier.
